# EUROPÄISCHE PATENTSCHRIFT

(11) **EP 2 297 101 B1**
(45) Veröffentlichungstag und Bekanntmachung des Hinweises auf die Patenterteilung: **13.07.2016**
(21) Anmeldenummer: 09749790.3
(22) Anmeldetag: 18.05.2009
(51) Int. Cl.: C07F 3/00, C07D 211/12

(54) **VERFAHREN ZUR HERSTELLUNG VON MAGNESIUMAMIDEN**
METHOD FOR MANUFACTURING MAGNESIUM AMIDES
PROCÉDÉ POUR PRODUIRE DES AMIDES DE MAGNÉSIUM

(30) Priorität: 21.05.2008 DE 102008001905
(43) Veröffentlichungstag der Anmeldung: 23.03.2011
(73) Patentinhaber: Chemetall GmbH, 60487 Frankfurt (DE)
(72) Erfinder: MURSO, Alexander, 60489 Frankfurt (DE); KURTH, Christopher, 64839 Münster (DE); RITTMEYER, Peter, 65843 Sulzbach/Taunus (DE)
(74) Vertreter: Rottmayer, Hans
(86) Internationale Anmeldenummer: PCT/EP2009/055987
(87) Internationale Veröffentlichungsnummer: WO 2009/141300

(56) Entgegenhaltungen:
- EP-A- 1 207 153
- EP-A1- 1 810 974
- WO-A-99/46224

## Beschreibung

Die vorliegende Erfindung betrifft ein Verfahren zur Herstellung von Amidomagnesiumhalogeniden, sowie deren Abmischung mit Alkalimetallsalzen in aprotischen, organischen Lösemitteln, die nach diesem Verfahren erhältlichen Verbindungen und deren Anwendung in der Synthesechemie, beispielsweise zur Deprotonierung von enolisierbaren Systemen, funktionalisierten Aromaten und Heteroaromanten.

Magnesiumamide der allgemeinen Form (R'R"NMgX) sowie deren Abmischung mit Alkalimetallsalzen der Form MY finden in der Synthesechemie breite Anwendung. Gegenüber Lithiumamiden oder Organolithiumverbindungen zeigen sie eine geringere Nucleophilie, wodurch höhere Selektivitäten und Ausbeuten erreicht werden. Sie werden häufig in Deprotonierungsreaktionen an enolisierbaren Systemen oder an funktionalisierten Aromaten und Heteroaromaten eingesetzt. In Gegenwart von Alkalimetallsalzen, beispielsweise LiCl, zeigen sie eine erhöhte kinetische Basizität, wodurch Reaktionszeiten deutlich verkürzt werden. Die Verwendung von Amidomagnesiumhalogeniden erlaubt zudem Synthesen unter nicht kryogenen Bedingungen. Zudem erlauben sie aufgrund anderer Regioselektivitäten im Vergleich zu ähnlichen Reagenzien, wie beispielsweise Lithiumamiden, die Herstellung von Intermediaten die auf anderem Weg nicht oder nur über mehrere Syntheseschritte erhalten werden können (A. Krasovskiy, V. Krasovskaya, P. Knochel, Angew. Chem. 2006, 118, 3024; b) W. Lin, O. Baron, P. Knochel, Org. Lett. 2006, 8, 5673; c) G. C. Clososki, C. J. Rohbogner, P. Knochel, Angew. Chem. 2007, 119, 7825; d) P. E. Eaton, R. M. Martin, J. Org. Chem. 1988, 53, 2728, e) Y. Kondo, A. Yoshida, T. Sakamoto, J. Chem. Soc. Perkin Trans. 1, 1996, 2331 und darin zitierte Literatur und EP 1810974 A1).

Amidomagnesiumhalogenide (R'R"NMgX) werden im Allgemeinen durch Umsetzung von Grignardreagenzien (R¹MgX) mit den entsprechenden Aminen (R'R"NH) erhalten. Die Amine (R'R"NH) reagieren dabei mit der Grignardverbindung (R¹MgX) zunächst unter Ausbildung eines Elektronen-Donor-Akzeptor-Komplexes, der sich dann unter Eliminierung von R¹-H zum Amidomagnesiumhalogenid (R'R"NMgX) umwandelt (Methoden der Organischen Chemie (Houben-Weyl), E. Müller (Eds.), Band XIII/2a, 4. Auflage, Thieme Verlag, Stuttgart, 1973 und darin zitierte Literatur und F. C. Frostick Jr., C. R. Hauser, J. Am. Chem. Soc. 1949, 71, 1350).

Mischungen von Amidomagnesiumhalogeniden (R'R"NMgX) mit Alkalimetallsalzen MY können durch Umsetzung von Grignardverbindungen mit Aminen und nachträglicher Zugabe des Alkalimetallsalzes erhalten werden oder durch Zugabe des Alkalimetallsalzes zur Grignardverbindung und anschließender Umsetzung mit einem Amin (Abbildung 1).

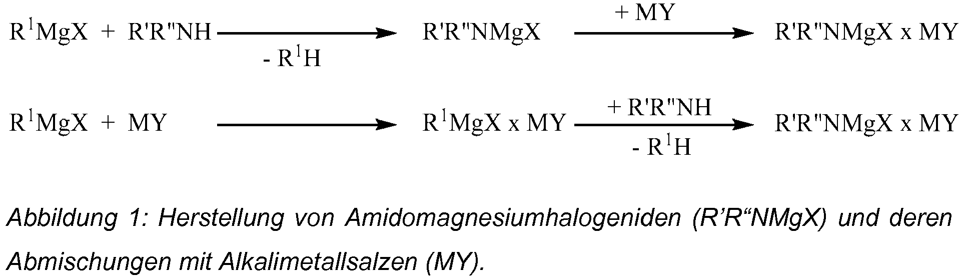

Letzterer Weg ist beispielsweise von Knochel et al beschrieben. In EP 1810974 A1 wird über die Herstellung und Verwendung von beispielsweise TMPMgCl/LiCl, ca. 1 M in THF (TMP = 2,2,6,6-Tetramethylpiperidino) berichtet. Die Herstellung erfolgt durch Umsetzung von Turbogrignardreagenzien, beispielsweise iso-PrMgCl/LiCl, ca. 14% in THF, und TMPH (2,2,6,6-Tetramethylpiperdin) gem. Abbildung 2.

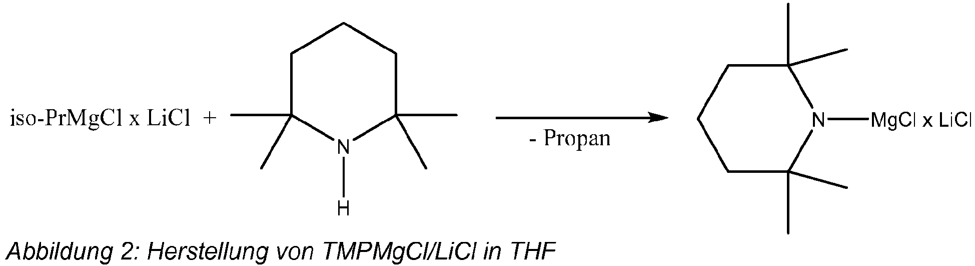

Alle bisher bekannten Wege zur Herstellung von Amidomagnesiumhalogeniden (R'R"NMgX) sowie deren Abmischungen mit Alkalimetallsalzen erfordern in einem ersten Schritt immer die Herstellung einer geeigneten Grignardverbindung (R¹MgX) oder deren Mischung mit Alkalimetallsalz gefolgt von einer Umsetzung der gewonnenen Grignardverbindung mit einem entsprechenden Amin und ggf. eine nachträgliche Einbringung des Alkalimetallsalzes. Die bekannten Verfahren zur Herstellung von Amidomagnesiumhalogeniden (R'R"MgX) oder deren Mischungen mit Alkalimetallsalzen (MY) stellen damit mindestens zweistufige, aufwändige und technisch anspruchsvolle Prozesse dar.

Aufgabe der vorliegenden Erfindung ist es daher, ein Verfahren bereitzustellen, das die Nachteile des Standes der Technik überwindet. Insbesondere lag der vorliegenden Erfindung zugrunde ein einfaches, einstufiges und wirtschaftliches Verfahren zur Herstellung von Amidomagnesiumhalogeniden (R'R"NMgX) sowie deren Abmischung mit Alkalimetallsalzen (MY) zu entwickeln.

Erfindungsgemäß wird die Aufgabe überraschenderweise wie folgt gelöst.

Das erfindungsgemäße Verfahren liefert eine Vielzahl an Amidomagnesiumhalogeniden (R'R"NMgX). Das erfindungsgemäße Verfahren liefert gleichartig oder verschiedenartig substituierte Amidomagnesiumhalogeniden (R'R"NMgX) in nur einem definierten Lösemittel oder in definierten Lösemittelgemischen. Zusätzlich liefert das erfindungsgemäße Verfahren alkalisalzhaltige oder -freie Lösungen von Amidomagnesiumhalogeniden (R'R"NMgX) in definierten Lösemitteln oder Lösemittelgemischen. Die erfindungsgemäß hergestellten Amidomagnesiumhalogenide (R'R"NMgX) sowie deren Mischungen mit Alkalimetallsalzen MY können zur Herstellung zahlreicher zum Teil hochfunktionalisierter Stoffe, wie Pharmazeutika, Naturstoffderivate, Polymermaterialien, Agrochemikalien, Spezialitäten und Katalysatoren verwendet werden, beispielsweise in Deprotonierungsreaktionen und zur Herstellung von Zinkamiden.

Überraschenderweise wurde gefunden, dass durch Umsetzung von Magnesium mit einem organischen Halogenid (R¹X) in einem aprotischen organischen Lösemittel in Gegenwart eines protischen Amins (R'R"NH) direkt die gewünschten Amidomagnesiumhalogenide (R'R"NMgX) in hohen Ausbeuten und hoher Reinheit gebildet werden. Zur Herstellung von Mischungen aus Amidomagnesiumhalogeniden (R'R"MgX) und Alkalimetallsalzen (MY) wird die Reaktion vorzugsweise direkt in Gegenwart des Alkalimetallsalzes durchgeführt. Die Einbringung des Alkalimetallsalzes (MY) kann jedoch auch erst nach erfolgter Umsetzung erfolgen.

Die Bildung der gewünschten Amidomagnesiumhalogenide (R'R"NMgX) zeigt, dass während der Reaktion eine Grignardverbindung (R¹MgX) entsteht, die dann direkt durch das protische Amin (R'R"NH) abgefangen wird, wodurch sich die gewünschten Amidomagnesiumhalogenide (R'R"NMgX) unter Eliminierung von R¹-H bilden.

Die Konkurrenzreaktion des Amins mit Magnesium würde zur Bildung von Wasserstoff (H₂) und einem Magnesiumamid der Form (R'R"N)₂Mg führen. Dies wird jedoch im erfindungsgemäßen Verfahren nicht beobachtet.

Die Bildung von Amidomagnesiumhalogeniden (R'R"NMgX) nach dem erfindungsgemäßen Verfahren steht im Gegensatz zu den Erwartungen, dass in Gegenwart von protischen Verbindungen, wie beispielsweise Wasser, Alkoholen oder Aminen die Bildung von Grignardverbindungen (R¹MgX) sehr stark gehindert ist, was dazu führt, dass die Grignardbildung überhaupt nicht oder erst nach Zugabe erheblicher Mengen an Halogenid (R¹X) initiiert wird. So ist beispielsweise beschrieben, dass zur Herstellung von Grignardverbindungen alle verwendeten Reagenzien und Apparaturen trocken sein müssen und am besten weniger als 0,02 Gew.-% Wasser enthalten sollten (Handbook of Grignard Reagents, G. S. Silverman, P. E. Rakita (Eds.), Marcel Dekker, Inc., New York, 1996). Wird die Grignardbildung erst nach Zugabe erheblicher Mengen an organischem Halogenid (R¹X) initiiert, läuft als Nebenreaktion vermehrt eine Wurtzkupplung durch Reaktion des akkumulierten, organischen Halogenides (R¹X) mit gebildetem Grignardreagenz (R¹MgX) ab, was zur Bildung erheblicher Mengen an unerwünschtem Nebenprodukt (R¹-R¹) führt.

Bekannt ist die Verwendung von tertiären Aminen (R₃N) als Lösemittel in der Herstellung und Anwendung von Grignardverbindungen. Diese bilden mit GrignardReagenzien Elektronen-Donor-Akzeptor-Komplexe. Werden gleichzeitig in der

Grignardherstellung oder der Anwendung von Grignardverbindungen noch andere Donorsolventien, beispielsweise etherische Lösemittel verwendet, so können tertiäre Amine je nach Donorstärke das etherische Lösemittel verdrängen. Damit beeinflussen sie die Aggregation, Löslichkeit und auch die Reaktivität von Grignardverbindungen.

Die Erfindung wird anhand folgender allgemeiner Abbildung 3 erläutert.

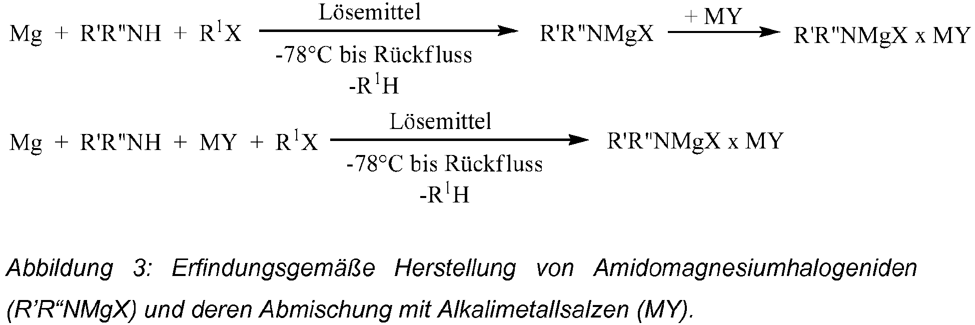

Das erfindungsgemäße Verfahren wird allgemein wie folgt durchgeführt:
Magnesium wird in einem aprotischen organischen Lösemittel oder Lösemittelgemisch und einem protischen Amin (R'R"NH) vorgelegt und mit einem Halogenid (R¹X) zur Reaktion gebracht.

Vorzugsweise wird Magnesium in einem aprotischen organischen Lösemittel oder Lösemittelgemisch und einem Amin (R'R"NH) vorgelegt und ein organisches Halogenid (R¹X) zu dieser Mischung dosiert.

In einer weiteren Ausführungsform wird Magnesium in einem aprotischen organischen Lösemittel oder Lösemittelgemisch vorgelegt und ein protisches Amin (R'R"NH) und ein organisches Halogenid (R¹X) als Mischung oder einzeln parallel dosiert.

Zur Herstellung von Mischungen von R'R"NMgX mit Alkalisalzen (MY) wird vorzugsweise das Alkalimetallsalz mit vorgelegt.

Die Menge an gelöstem Alkalimetallsalz in der Produktlösung richtet sich dabei nach dem angestrebten molaren Verhältnis von Alkalisalz (MY) zu Produkt R'R"NMgX, der Konzentration an Produkt R'R"NMgX und dem Lösemittel oder Lösemittelgemisch. Mit zunehmender Produktkonzentration fällt die Löslichkeit des Alkalimetallsalzes und damit auch das molare Verhältnis von MY zu R'R"NMgX. Desgleichen fällt die Löslichkeit an Alkalisalz mit zunehmendem Gehalt an unpolaren Lösemitteln. Die Produktkonzentration R'R"NMgX beträgt zwischen 5 und 80 Gew.-%, bevorzugt zwischen 5 und 50 Gew.-%.

Bevorzugt wird ein Überschuss von 0 - 50% an Alkalisalz bezogen auf das gewünschte molare Verhältnis von MY zu R'R"NMgX eingesetzt, vorzugsweise zwischen 0 - 20%, besonders bevorzugt zwischen 0 - 10%.

Werden molare Verhältnisse von MY zu R'R"NMgX ≥ 1 angestrebt beträgt der Überschuss von Alkalisalz (MY) zu organischem Halogenid (R¹X) zwischen 0 - 50%, bevorzugt zwischen 0 - 20%.

Als Magnesium können Mg-Blöcke, -Späne, -Raspeln, -Granulat oder auch Mg-Pulver verwendet werden, vorzugsweise werden Mg-Späne, -Raspeln, -Granulat oder -Pulver verwendet.

Das Magnesium kann mit Methoden des Standes der Technik vor Beginn der Umsetzung zur Minimierung von Nebenreaktionen, wie beispielsweise Wurtz-Reaktion, aktiviert werden, beispielsweise durch Zugabe von Iod, 1,2-Dibrommethan, Trimethylsilylchlorid, zuvor hergestellter Grignardlösung (R¹MgX) oder zuvor hergestelltem Produkt R'R"NMgX oder deren Abmischungen mit einem Alkalisalz (MY). Vorzugsweise wird mit zuvor hergestellter Grignardlösung (R¹MgX) oder zuvor hergestelltem Produkt R'R"NMgX oder deren Abmischungen mit einem Alkalisalz (MY) aktiviert. Die Menge an Grignardlösung (R¹MgX) oder zuvor hergestelltem Produkt R'R"NMgX oder deren Abmischungen mit einem Alkalisalz (MY) zur Aktivierung beträgt zwischen 0,01 und 50 mol% bezogen auf die vorgelegte Magnesiummenge, bevorzugt zwischen 0,1 und 10 mol%.

Die Verfahren werden zwischen -78°C und Rückflusstemperatur der Reaktionssuspension, vorzugsweise zwischen 0°C und Rückflusstemperatur durchgeführt.

Der Überschuss an Magnesium in Bezug auf das organische Halogenid (R¹X) beträgt zwischen 0 und 300%, bevorzugt zwischen 10 und 200%.

Der molare Verhältnis von protischem Amin (R'R"NH) zu organischen Halogenid (R¹X) beträgt zwischen 0,7 bis 1,5, vorzugsweise zwischen 0,9 und 1,3, besonders bevorzugt zwischen 1,0 und 1,2.

Zur Herstellung von R'R"NMgX-Lösungen sowie deren Mischungen mit Alkalisalz (MY) in Mischungen aus Ethern und Kohlenwasserstoffen werden vorzugsweise organische Halogenide (R¹X) verwendet, die während der Reaktion den gewünschten Kohlenwasserstoff produzieren, beispielsweise Pentylchlorid oder - bromid, Hexylchlorid oder -bromid oder deren Isomere, Cyclohexylchlorid oder - bromid, Phenylchlorid oder -bromid oder Benzylchlorid oder -bromid.

Zur Herstellung von R'R"NMgX-Lösungen oder Mischungen mit Alkalisalz (MY) in reinen Ethern werden vorzugsweise organische Halogenide (R¹X) verwendet, die während der Reaktion flüchtige Gase bilden, beispielsweise, Methylchlorid oder - bromid, Ethylchlorid oder -bromid, n- oder iso-Propylchlorid oder -bromid oder n-, sek- oder tert-Butylchlorid oder -bromid.

Zur Aufarbeitung der Reaktionsmischung werden gängige Methoden des Standes der Technik verwendet, vorzugsweise wird über ein geeignetes Filter filtriert oder nach Absitzen vom noch vorhandenen Feststoffe abgehebert.

Wird abgehebert, verbleibt vorzugsweise ein Rest an Reaktionsmischung im Reaktor. Dieser Rest kann in Folgeansätzen mit verwendet werden. Er enthält aktives Magnesium, so dass für Folgeansätze keine weitere Aktivierung des Magnesiums nötig ist.

Alkalimetallsalze im Sinne der Erfindung sind Verbindungen der Allgemeinen Formel MY,
- wobei M Lithium, Natrium und Kalium ist, bevorzugt Lithium und Kalium, besonders bevorzugt Lithium
- und wobei Y ausgewählt ist aus Chlorid, Bromid, Iodid oder OZ,
- wobei OZ ein Alkoholat-Anion darstellt und Z gewählt ist aus einem organischen, verzweigten oder unverzweigten, gesättigten oder ungesättigten, aliphatischen oder aromatischen Kohlenstofffragment, das zwischen einem und 20 Kohlenstoffatome enthält oder gewählt ist aus R²R³R⁴Si, wobei R², R³ und R⁴ wie unten stehend definiert sind,
- wobei Fragmente mit einem bis 10 Kohlenstoffatome und R²R³R⁴Si bevorzugt sind,
- wobei Methyl, Ethyl, n-Propyl, iso-Propyl, n-Butyl, sek-Butyl, tert-Butyl, tert-Amyl, Phenyl und R²R³R⁴Si besonders bevorzugt sind.

Insbesondere bevorzugt an MY verwendet werden Lithiumchlorid, Lithiumbromid, Lithium-methoxid, Lithium-ethoxid, Lithium-n-propoxid, Lithium-iso-propoxid, Lithium-tert-butoxid, Lithium-tert-amoxid, Lithium-trimethysilyloxid, Lithium-tri-tert-butylsilyloxid.

In den organischen Halogeniden (R¹X), den Grignardverbindungen (R¹MgX) und den Produkten R'R"NMgX ist X gewählt aus Chlor, Brom oder Iod, bevorzugt aus Chlor und Brom, besonders bevorzugt ist Chlor.

In den organischen Halogeniden (R¹X), den protischen Aminen (R'R"NH), den Grignardverbindungen (R¹MgX) und den Silylfragmenten (R²R³R⁴Si) sind R¹, R², R³, R⁴, R' und R" unabhängig voneinander gewählt aus:
- gesättigten, ungesättigten, verzweigten, unverzweigten, funktionalisierten, unfunktionalisierten, aliphatischen, cyclischen, heterozyklischen oder aromatischen organischen Fragmenten wobei,
- gesättigte, ungesättigte, verzweigte, unverzweigte, funktionalisierte, unfunktionalisierte, aliphatische, cyclische, heterozyklische oder aromatische organische Fragmente mit 1 bis 20 Kohlenstoffatomen bevorzugt sind, wobei
- insbesondere Methyl-, Ethyl-, n-Propyl-, iso-Propyl, n-Butyl-, sek-Butyl, tert-Butyl, Pentyl-, Hexyl-, Heptyl-, Octyl-, Cyclohexyl, Phenyl und Benzyl-Fragmente besonders bevorzugt sind und wobei
- an organischen Halogeniden (R¹X) insbesondere bevorzugt sind Methylchlorid oder -bromid, Ethylchlorid oder -bromid, n- oder iso-Propylchlorid oder -bromid, n-, sek- oder tert-Butylchlorid oder -bromid, n-, iso-, sek- oder tert-Pentylchlorid oder -bromid, n-Hexylchlorid oder -bromid, Chlorbenzol oder Brombenzol oder Benzylchlorid oder -bromid und wobei
- an protischen Aminen (R'R"NH) insbesondere bevorzugt sind Dimethylamin, Diethylamin, Di-n-Propylamin, Di-iso-propylamin (DIPA), Di-n-butylamin, Di-sekbutylamin, Di-tert-butylamin, Dicyclohexylamin, N-tert-Butyl-iso-propylamin, Hexamethyldisilazan, Piperidin und 2,2,6,6-Tetramethylpiperidin (TMP).

Als aprotische, organische Lösemittel bevorzugt sind aliphatische oder aromatische Kohlenwasserstoffe, Heterozyklen, Ether, oder Mischungen daraus, besonders bevorzugt sind Ether oder Mischungen aus Ethern und aliphatischen oder aromatischen Kohlenwasserstoffe, insbesondere bevorzugt sind Ether.

Unter den Begriff aliphatische Kohlenwasserstoffe fallen cyclische, gesättigte, ungesättigte, verzweigte und unverzweigte Kohlenwasserstoffe. Bevorzugt werden gesättigte oder cyclische, verzweigte oder unverzweigte Kohlenwasserstoffe mit 5 bis 20 Kohlenstoffatomen eingesetzt, besonders bevorzugt n-Pentan, n-Hexan, n-Heptan, n-Octan oder deren Isomere, Cyclopentan, Cyclohexan und Methylcyclohexan.

Unter den Begriff Ether fallen acyclische, cyclische, gesättigte, ungesättigte, verzweigte, unverzweigte, gleichartig substituierte und unterschiedlich substituierte Ether mit mindestens einem Sauerstoffatom, bevorzugt mit einem bis vier Sauerstoffatomen. Weiterhin bevorzugt als Ether sind Dimethylether, Diethylether, Dibutylether, Dimethoxyethan, Diethoxymethan, Polyethylenglykol, Methyl-tert-butylether, Cyclopentylmethylether, Dioxan, Tetrahydrofuran (THF) und 2-Methyltetrahydrofuran (2-Methyl-THF) und besonders bevorzugt sind THF und 2-Methyl-THF und Cyclopentylmethylether.

Unter den Begriff aromatische Kohlenwasserstoffe fallen unsubstituierte, einfach substituierte und mehrfach substituierte aromatische Verbindungen. Bevorzugt eingesetzt werden Benzol, Toluol, Ethylbenzol, Cumol und Xylol, sowie deren Isomere.

Gegenstand der Erfindung im Einzelnen ist:
- ein Verfahren zur Herstellung von Amidomagnesiumhalogeniden (R'R"NMgX) in aprotischen, organischen Lösemitteln, dadurch gekennzeichnet dass zur Herstellung aprotische organische Lösemittel, Magnesium, protische Amine (R'R"NH) und organische Halogenide (R¹X) verwendet werden;
- ein Verfahren zur Herstellung von Mischungen aus Amidomagnesiumhalogeniden (R'R"NMgX) mit Alkalimetallsalzen (MY) in aprotischen, organischen Lösemitteln, dadurch gekennzeichnet dass zusätzlich noch Alkalimetallsalze (MY) verwendet werden;
- die nach dem erfindungsgemäßen Verfahren hergestellten Verbindungen;
- die Verwendung der nach dem erfindungsgemäßen Verfahren hergestellten Verbindungen in der Synthesechemie;
- die Verwendung der nach dem erfindungsgemäßen Verfahren hergestellten Verbindungen zur Deprotonierung von enolisierbaren Systemen;
- die Verwendung der nach dem erfindungsgemäßen Verfahren hergestellten Verbindungen zur Deprotonierung von funktionalisierten Aromaten und Heteroaromaten.

### Beispiele:

Die Erfindung wird anhand der folgenden Beispiele erläutert, ohne sie darauf einzuschränken. In dem Bereich der Chemie geschulte Fachkräfte werden erkennen, dass diese Beispiele auch andere hier nicht angeführten Vorgehensweisen aufzeigen, um die erfindungsgemäß hergestellten Amidomagnesiumhalogeniden (R'R"NMgX) und deren Abmischung mit Alkalimetallsalzen (MY) zu erhalten. Daher sind viele Variationen und Modifikationen möglich, die Gegenstand der vorliegenden Erfindung sind.

Alle Versuche wurden in einer Argon-Atmosphäre unter Verwendung von Schlenk-Techniken durchgeführt. Es wurden technische Rohstoffe eingesetzt. An Magnesium wurden Mg-Raspeln eingesetzt, jedoch können erfindungsgemäß auch andere zur Grignardherstellung geeigneten Magnesiumqualitäten verwendet werden.

Zur Verdeutlichung der Effizienz des Verfahrens wurde die Herstellung von TMPMgCl/LiCl in THF und in THF/Toluol, von TMPMgCl in THF/Toluol sowie von DIPAMgCl/LiCl in THF/Toluol gewählt. Zur Herstellung von THF-Lösungen wurde als organisches Halogenid (R¹X) iso-Propylchlorid verwendet, zur Herstellung von THF/Toluol-Lösungen Benzylchlorid. Es ist bekannt, dass organische Bromide (R¹Br) im Allgemeinen besser zur Grignardherstellung geeignet sind als die entsprechenden Chloride (R¹Cl). Somit unterstreicht die getroffene Wahl die Allgemeinheit des Verfahrens. Als Alkalimetallsalz wurde LiCl gewählt, jedoch können auch andere im Medium lösliche Alkalimetallsalze verwendet werden.

Die Beispiele werden im Folgenden erläutert. Die genauen Ansatzmengen, Überschüsse Reaktions- und Dosierzeiten, sowie Reaktions- und Dosiertemperaturen sind in den Tabellen 1, 3 und 5 angeführt. Die Analysenergebnisse und Auswertung der Versuche sind inklusive der Ausbeuten in den Tabellen 2, 4 und 6 angeführt.

Der genaue Gehalt und die Zusammensetzung der Chargen wurden analytisch bestimmt. Der Gehalt an Magnesium wurde komplexometrisch, der Gehalt an Lithium flammenemissionsspektroskopisch, der Gehalt an Chlorid argentometrisch, der Gehalt an Totalbase acidimetrisch und der Amin-Gehalt nach Kjeldahl nach Hydrolyse bestimmt. Die Aktivbase wurde nach Watson-Eastham bestimmt.

Durch GC/MS-Untersuchungen nach Derivatisierung und ¹H- und ¹³C-NMR-Messungen wurde die Identität und Reinheit der isolierten Produkte bestätigt.

### Beispiel 1: Herstellung von TMPMgCl/LiCl in THF/Toluol aus Mg, LiCl, TMPH und Benzylchlorid mit Aktivierung des Magnesiums mit TMPMgCl/LiCl in THF/Toluol

Magnesium, THF, Lithiumchlorid, TMPMgCl/LiCl in THF/Toluol werden in einem Doppelmantelreaktor mit Rührer, Dosierstation und Rückflußkühler suspendiert und 30 Minuten bei Raumtemperatur gerührt. Anschließend wird die Gesamtmenge an TMPH schnell in einer Portion zugegeben. Dann wurden ca. 5 mol% der Gesamtmenge an Benzylchlorid schnell zugegeben. An einem sofortigen, deutlichen Temperaturanstieg konnte die Initiierung der Reaktion sichergestellt werden. Die Restmenge an Benzylchlorid wurde kontinuierlich dosiert. Nach Nachreaktion wurde die Lösung filtriert. Es wurde eine bräunliche, klare Lösung erhalten.

### Beispiel 2: Herstellung von TMPMgCl in THF/Toluol aus Mg, TMPH und Benzylchlorid mit Aktivierung des Magnesiums mit TMPMgCl in THF/Toluol

Magnesium, THF, TMPMgCl in THF/Toluol werden in einem Doppelmantelreaktor mit Rührer, Dosierstation und Rückflußkühler suspendiert und 30 min bei Raumtemperatur gerührt. Anschließend wurde die Gesamtmenge an TMPH schnell in einer Portion zugegeben. Dann wurden ca. 5 mol% der Gesamtmenge an Benzylchlorid schnell zugegeben. An einem sofortigen, deutlichen Temperaturanstieg konnte die Initiierung der Reaktion sichergestellt werden. Die Restmenge an Benzylchlorid wurde kontinuierlich dosiert. Nach Nachreaktion wurde die Lösung filtriert. Es wurde eine bräunliche, klare Lösung erhalten.

### Beispiel 3: Herstellung von TMPMgCl/LiCl in THF aus Mg, LiCl, TMPH und iso-PrCl mit Aktivierung des Magnesiums mit iso-PrMgCl in THF

Magnesium, THF, Lithiumchlorid und iso-PrMgCl in THF werden in einem Doppelmantelreaktor mit Rührer, Dosierstation und Rückflußkühler suspendiert und 30 min bei Raumtemperatur gerührt. Die Gesamtmenge an TMPH wurde schnell in einer Portion zugegeben. In der Gesamtmenge an TMPH ist die Menge die zur Reaktion von iso-PrMgCl mit TMPH zum gewünschten Produkt gem. Abbildung 1 nötig ist berücksichtigt. Es wurden ca. 5 mol% der Gesamtmenge an iso-PrCl schnell zugegeben. An einem sofortigen, deutlichen Temperaturanstieg konnte die Initiierung der Reaktion sichergestellt werden. Die Restmenge an iso-PrCl wurde kontinuierlich dosiert. Nach Nachreaktion wurde die Lösung filtriert. Es wurde eine bräunliche, klare Lösung erhalten.

### Beispiel 4: Herstellung von DIPAMgCl/LiCl in THF/Toluol aus Mg, LiCl, DIPAH und Benzylchlorid ohne Aktivierung des Magnesiums

Magnesium, THF, Lithiumchlorid und die Gesamtmenge an DIPAH werden in einem Doppelmantelreaktor mit Rührer, Dosierstation und Rückflußkühler vorgelegt. Es wurden ca. 5 mol% der Gesamtmenge an Benzylchlorid schnell zugegeben. An einem sofortigen, deutlichen Temperaturanstieg konnte die Initiierung der Reaktion sichergestellt werden. Die Restmenge an Benzylchlorid wurde kontinuierlich dosiert. Nach Nachreaktion wurde die Lösung filtriert. Es wurde eine bräunliche, klare Lösung erhalten.

**Tabelle 1: Herstellparameter für TMPMgCl/LiCl in THF/Toluol und TMPMgCl in THF/Toluol**

| **Beispiel** | **TMPH** | | **BC¹** | | **Mg** | | **LiCl** | | **THF** | **Mg-Aktivierung** | | **Temp.** | **Dosierzeit** | **Nachreaktion** | |
|---|---|---|---|---|---|---|---|---|---|---|---|---|---|---|---|
| | **g** | **mmol** | **g** | **mmol** | **g** | **mmol** | **g** | **mmol** | **g** | **mmol** | **Stoff** | **°C** | **min** | **min** | **°C** |
| **1** | 85,91 | 608,17 | 67,22 | 531,01 | 26,8 | 1102,4 | 29,45 | 694,74 | 287,8 | 20,90 | TMPMgCl² | 25 | 240 | 120 | 25 |
| **2** | 78,00 | 552,17 | 66,95 | 528,87 | 24,9 | 1024,3 | - | - | 515,2 | 21,50 | TMPMgCl³ | 25 | 300 | 120 | 25 |

| | | | | | | | | | | | | | | | |
|---|---|---|---|---|---|---|---|---|---|---|---|---|---|---|---|
| ¹ BC = Benzylchlorid; ² TMPMgCl/LiCl in THF/Toluol: Aktivbase AB = 0,96 mmol/g; ³ TMPMgCl in THF/Toluol: Aktivbase AB = 0,66 mmol/g. | | | | | | | | | | | | | | | |

**Tabelle 2: Ergebnisse zu den Beispielen aus Tabelle 1**

| **Beispiel** | **OH** | **Mg** | **C1** | **Li** | **TMPH** | **AB¹** | **LiCl²** | **Verhältnis** | **Ausbeute** ³ | | | **Konzentration** |
|---|---|---|---|---|---|---|---|---|---|---|---|---|
| | **mmol**/**g** | | | | | | **mmol**/**g** | **LiCl : AB** | **% AB⁴** | **% LiCl⁵** | **% TMPH**⁵ | **Gew.-%** |
| **1** | 2,30 | 1,10 | 2,18 | 1,03 | 1,23 | 1,05 | 1,03 | 0,98 | 91,3 | 68,5 | 93,4 | 21,0 |
| **2** | 1,50 | 0,75 | 0,77 | - | 0,77 | 0,71 | - | - | 88,6 | - | 92,1 | 13,8 |

| | | | | | | | | | | | | |
|---|---|---|---|---|---|---|---|---|---|---|---|---|
| ¹ AB = Aktivbase; ² über Li; ³ isolierte Ausbeute ohne Waschlösungen; ⁴ bezogen auf Menge Benzylchlorid BC; ⁵ bezogen auf Einsatzmenge. | | | | | | | | | | | | |

**Tabelle 3: Herstellparameter für TMPMgCl/LiCl in THF**

| **Beispiel** | **TMPH** | | **iso-PrCl** | | **Mg** | | **LiCl** | | **THF** | **Mg**-**Aktivierung** | | **Temp**. | **Dosierzeit** | **Nachreaktion** | |
|---|---|---|---|---|---|---|---|---|---|---|---|---|---|---|---|
| | **g** | **mmol** | **g** | **mmol** | **g** | **mmol** | **g** | **mmol** | **g** | **mmol** | **Stoff** | **°C** | **min** | **min** | **°C** |
| **3** | 89,30 | 632,17 | 44,80 | 570,41 | 16,0 | 659,8 | 25,34 | 597,78 | 340,7 | 56,80 | iso-PrMgCl¹ | 45 | 200 | 90 | 45 |

| | | | | | | | | | | | | | | | |
|---|---|---|---|---|---|---|---|---|---|---|---|---|---|---|---|
| ¹ iso-PrMgCl in THF: Aktivbase AB = 1,33 mmol/g. | | | | | | | | | | | | | | | |

**Tabelle 4: Ergebnisse zu den Beispielen aus Tabelle 3**

| **Beispiel** | **OH** | **Mg** | **Cl** | **Li** | **TMPH** | **AB¹** | **LiCl²** | **Verhältnis** | **Ausbeute³** | | | **Konzentration** |
|---|---|---|---|---|---|---|---|---|---|---|---|---|
| | **mmol**/**g** | | | | | | **mmol**/**g** | **LiCl: AB** | **%AB⁴** | **% LiCl⁵** | **% TMPH**⁵ | **Gew**.-% |
| **3** | 2,33 | 1,08 | 2,19 | 1,11 | 1,23 | 1,07 | 1,11 | 1,04 | 92,4 | 91,4 | 95,8 | 21,4 |

| | | | | | | | | | | | | |
|---|---|---|---|---|---|---|---|---|---|---|---|---|
| ¹ AB = Aktivbase; ² über Li; ³ isolierte Ausbeute ohne Waschlösungen; ⁴ bezogen auf Menge iso-PrCl; ⁵ bezogen auf Einsatzmenge. | | | | | | | | | | | | |

**Tabelle 5: Herstellparameter für DIPAMgCl/LiCl in THF/Toluol**

| **Beispiel** | **DIPAH**¹ | | **BC²** | | **Mg** | | **LiCl** | | **THF** | **Mg**-**Aktivierung** | | **Temp**. | **Dosierzeit** | **Nachreaktion** | |
|---|---|---|---|---|---|---|---|---|---|---|---|---|---|---|---|
| | **g** | **mmol** | **g** | **mmol** | **g** | **mmol** | **g** | **mmol** | **g** | **mmol** | **Stoff** | **°C** | **min** | **min** | **°C** |
| **4** | 69,27 | 684,55 | 82,67 | 653,05 | 33,5 | 1375,98 | 30,10 | 710,07 | 772,9 | - | - | 25-35 | 260 | 25 | 100 |

| | | | | | | | | | | | | | | | |
|---|---|---|---|---|---|---|---|---|---|---|---|---|---|---|---|
| ¹ DIPAH = Di-iso-propylamin; ² BC = Benzylchlorid. | | | | | | | | | | | | | | | |

**Tabelle 6: Ergebnisse zu den Beispielen aus Tabelle 5**

| **Beispiel** | **OH** | **Mg** | **Cl** | **Li** | **DIPAH** | **AB¹** | **LiCl²** | **Verhältnis** | **Ausbeute³** | | | **Konzentration** |
|---|---|---|---|---|---|---|---|---|---|---|---|---|
| | **mmol**/**g** | | | | | | **mmol**/**g** | **LiCl: AB** | **%AB⁴** | **% LiCl⁵** | **TMPH**⁵ | **Gew.-%** |
| **4** | 1,36 | 0,71 | 1,33 | 0,60 | 0,69 | 0,63 | 0,60 | 0,95 | 89,9% | 78,7% | 93,9% | 10,1 |

| | | | | | | | | | | | | |
|---|---|---|---|---|---|---|---|---|---|---|---|---|
| ¹ AB = Aktivbase; ² über Li; ³ isolierte Ausbeute ohne Waschlösungen; ⁴ bezogen auf Menge Benzylchlorid BC; ⁵ bezogen auf Einsatzmenge. | | | | | | | | | | | | |

## Patentansprüche

1. Verfahren zur Herstellung von Mischungen aus Amidomagnesiumhalogeniden der allgemeinen Formel R'R"NMgX mit Alkalimetallsalzen (MY), **dadurch gekennzeichnet,**
**dass** das Magnesium in einem aprotischen organischen Lösemittel oder Lösemittelgemisch und ein protisches Amin (R'R"NH) vorgelegt und ein organisches Halogenid (R¹X) zu dieser Mischung dosiert oder Magnesium in einem aprotischen organischen Lösemittel oder Lösemittelgemisch vorgelegt und ein protisches Amin (R'R"NH) und ein organisches Halogenid (R¹X) als Mischung oder einzeln parallel zu dosiert, wobei das Alkalimetallsalz mit vorgelegt wird
- an organischen Halogeniden (R¹X) Methylchlorid oder -bromid, Ethylchlorid oder -bromid, n- oder iso-Propylchlorid oder -bromid, n-, sek- oder tert-Butylchlorid oder -bromid, n-, iso-, sek- oder tert-Pentylchlorid oder -bromid, n-Hexylchlorid oder -bromid, Chlorbenzol oder Brombenzol oder Benzylchlorid oder -bromid und
- an protischen Aminen (R'R"NH) Dimethylamin, Diethylamin, Di-n-Propylamin, Di-iso-propylamin (DIPA), Di-n-butylamin, Di-sekbutylamin, Di-tert-butylamin, Dicyclohexylamin, N-tert-Butyl-iso-propylamin, Hexamethyldisilazan, Piperidin und 2,2,6,6-Tetramethylpiperidin (TMP) eingesetzt werden;
- wobei M Lithium, Natrium oder Kalium ist,
- und Y ausgewählt ist aus Chlorid, Bromid, Iodid oder OZ,
- wobei OZ ein Alkoholat-Anion darstellt und Z gewählt ist aus einem organischen, verzweigten oder unverzweigten, gesättigten oder ungesättigten, aliphatischen oder aromatischen Kohlenstofffragment, das zwischen einem und 20 Kohlenstoffatome enthält oder ausgewählt ist aus R²R³R⁴Si, wobei R², R³ und R⁴ unabhängig voneinander ausgewählt sind aus:
- gesättigten, ungesättigten, verzweigten, unverzweigten, funktionalisierten, unfunktionalisierten, aliphatischen, cyclischen, heterozyklischen oder aromatischen organischen Fragmenten mit 1 bis 20 Kohlenstoffatomen.

2. Verfahren nach Anspruch 1, **dadurch gekennzeichnet, dass** das Magnesium in Form von Mg-Blöcken, -Spänen, -Raspeln, -Granulat oder auch Mg-Pulver verwendet wird.

3. Verfahren nach Anspruch 2, **dadurch gekennzeichnet, dass** das Magnesium durch Zugabe von Iod, 1,2-Dibrommethan, Trimethylsilylchlorid, zuvor hergestellter Grignardlösung (R¹MgX) oder zuvor hergestelltem Produkt R'R"NMgX in Abmischungen mit einem Alkalisalz (MY), vorzugsweise mit zuvor hergestellter Grignardlösung (R¹MgX) oder zuvor hergestelltem Produkt R'R"NMgX in Abmischungen mit einem Alkalisalz (MY) aktiviert wird.

4. Verfahren nach Anspruch 43, **dadurch gekennzeichnet, dass** die Menge an Grignardlösung (R¹MgX) oder zuvor hergestelltem Produkt R'R"NMgX in Abmischungen mit einem Alkalisalz (MY) zur Aktivierung zwischen 0,01 und 50 mol% bezogen auf die vorgelegte Magnesiummenge, bevorzugt zwischen 0,1 und 10 mol% beträgt.

5. Verfahren nach mindestens einem der Ansprüche 1 bis 4, **dadurch gekennzeichnet, dass** das Magnesium in Bezug auf das organische Halogenid (R¹X) mit einem Überschuss zwischen 10 und 200% eingesetzt wird.

6. Verfahren nach mindestens einem der Ansprüche 1 bis 5, **dadurch gekennzeichnet, dass** das protische Amin (R'R"NH) und das organischen Halogenid (R¹X) im molaren Verhältnis von protischem Amin (R'R"NH) zu organischen Halogenid (R¹X) zwischen 0,7 bis 1,5, vorzugsweise zwischen 0,9 und 1,3, besonders bevorzugt zwischen 1,0 und 1,2 eingesetzt werden.

7. Verfahren nach mindestens einem der Ansprüche 1 bis 6, **dadurch gekennzeichnet, dass** in Mischungen aus Ethern und Kohlenwasserstoffen organische Halogenide (R¹X) verwendet werden, die während der Reaktion den gewünschten Kohlenwasserstoff produzieren.

8. Verfahren nach mindestens einem der Ansprüche 1 bis 6, **dadurch gekennzeichnet, dass** in reinen Ethern organische Halogenide (R¹X) verwendet werden, die während der Reaktion flüchtige Gase bilden.

9. Verfahren nach mindestens einem der Ansprüche 1 bis 8, **dadurch gekennzeichnet, dass** in den organischen Halogeniden (R¹X), den protischen Aminen (R'R"NH), den Grignardverbindungen (R¹MgX) und den Silylfragmenten (R²R³R⁴Si) sind R¹, R², R³, R⁴, R' und R" unabhängig voneinander gewählt aus:
- Methyl-, Ethyl-, n-Propyl-, iso-Propyl, n-Butyl-, sek-Butyl, tert-Butyl, Pentyl-, Hexyl-, Heptyl-, Octyl-, Cyclohexyl, Phenyl und Benzyl-Fragmente sind und wobei
- an organischen Halogeniden (R¹X) Methylchlorid oder -bromid, Ethylchlorid oder -bromid, n- oder iso-Propylchlorid oder -bromid, n-, sek- oder tert-Butylchlorid oder -bromid, n-, iso-, sek- oder tert-Pentylchlorid oder -bromid, n-Hexylchlorid oder -bromid, Chlorbenzol oder Brombenzol oder Benzylchlorid oder -bromid und wobei
- an protischen Aminen (R'R"NH) Dimethylamin, Diethylamin, Di-n-Propylamin, Di-iso-propylamin (DIPA), Di-n-butylamin, Di-sek-butylamin, Di-tert-butylamin, Dicyclohexylamin, N-tert-Butyl-iso-propylamin, Hexamethyldisilazan, Piperidin und 2,2,6,6-Tetramethylpiperidin (TMP) eingesetzt werden.

10. Verfahren nach Anspruch 1, **dadurch gekennzeichnet, dass** als Alkalimetallsalze Verbindungen der Allgemeinen Formel MY verwendet werden,
- wobei M Lithium und Kalium, besonders bevorzugt Lithium ist
- und wobei Y ausgewählt ist aus Chlorid, Bromid, Iodid oder OZ,
- wobei OZ ein Alkoholat-Anion darstellt und Z gewählt ist aus einem organischen, verzweigten oder unverzweigten, gesättigten oder ungesättigten, aliphatischen oder aromatischen Kohlenstofffragment, das zwischen einem und 20 Kohlenstoffatome enthält oder ausgewählt ist aus R²R³R⁴Si, wobei R², R³ und R⁴ wie unten stehend definiert sind,
- wobei Fragmente mit einem bis 10 Kohlenstoffatome und R²R³R⁴Si bevorzugt sind,
- wobei Methyl, Ethyl, n-Propyl, iso-Propyl, n-Butyl, sek-Butyl, tert-Butyl, tert-Amyl, Phenyl und R²R³R⁴Si besonders bevorzugt sind.

11. Verfahren nach Anspruch 10, **dadurch gekennzeichnet, dass** als Alkalimetallsalze Lithiumchlorid, Lithiumbromid, Lithium-methoxid, Lithium-ethoxid, Lithium-n-propoxid, Lithium-iso-propoxid, Lithium-tert-butoxid, Lithium-tert-amoxid, Lithium-trimethysilyloxid, Lithium-tri-tert-butylsilyloxid verwendet werden.

12. Verfahren nach Anspruch 1, **dadurch gekennzeichnet, dass** ein Überschuss von 0 - 50% an Alkalisalz bezogen auf das gewünschte molare Verhältnis von MY zu R'R"NMgX eingesetzt wird.

13. Verfahren nach Anspruch 1, **dadurch gekennzeichnet, dass** ein molares Verhältnis von MY zu R'R"NMgX ≥ 1 angestrebt wird, wobei der Überschuss von Alkalisalz (MY) zu organischem Halogenid (R¹X) zwischen 0 - 50% beträgt.

## Claims

1. A method for producing mixtures of amidomagnesium halides of the general formula R'R"NMgX with alkali-metal salts (MY), **characterised in that** the magnesium is placed in an aprotic organic solvent or solvent mix and a protic amine (R'R"NH), and an organic halide (R¹X) is metered into this mixture, or magnesium is placed in an aprotic organic solvent or solvent mix, and a protic amine (R'R"NH) and an organic halide (R¹X) are metered in as a mixture or individually in parallel, wherein the alkali-metal salt is also placed therewith,
- as regards organic halides (R¹X), methyl chloride or bromide, ethyl chloride or bromide, n- or iso-propyl chloride or bromide, n-, sec- or tert-butyl chloride or bromide, n-, iso-, sec- or tert-pentyl chloride or bromide, n-hexyl chloride or bromide, chlorobenzene or bromobenzene or benzyl chloride or bromide are employed, and
- as regards protic amines (R'R"NH), dimethylamine, diethylamine, di-n-propylamine, di-iso-propylamine (DIPA), di-n-butylamine, di-sec-butylamine, di-tert-butylamine, dicyclohexylamine, N-tert-butyl-iso-propylamine, hexamethyldisilazane, piperidine and 2,2,6,6-tetramethylpiperidine (TMP) are employed;
- wherein M is lithium, sodium or potassium,
- and Y is selected from chloride, bromide, iodide or OZ,
- wherein OZ represents an alcoholate anion, and Z is chosen from an organic, branched or unbranched, saturated or unsaturated, aliphatic or aromatic carbon fragment that contains between one and 20 carbon atoms or is selected from R²R³R⁴Si, where R², R³ and R⁴ are selected independently of one another from:
- saturated, unsaturated, branched, unbranched, functionalized, unfunctionalized, aliphatic, cyclic, heterocyclic or aromatic organic fragments with 1 to 20 carbon atoms.

2. A method according to claim 1, **characterised in that** the magnesium is used in the form of Mg blocks, chips, raspings, granules or even Mg powder.

3. A method according to claim 2, **characterised in that** the magnesium is activated by adding iodine, 1,2-dibromomethane, trimethylsilyl chloride, previously produced Grignard solution (R¹MgX) or previously produced product R'R"NMgX in admixtures with an alkali salt (MY), preferably with previously produced Grignard solution (R¹MgX) or previously produced product R'R"NMgX in admixtures with an alkali salt (MY).

4. A method according to claim 3, **characterised in that** the quantity of Grignard solution (R¹MgX) or previously produced product R'R"NMgX in admixtures with an alkali salt (MY) for the purposes of activation amounts to between 0.01 and 50 mol % relative to the quantity of magnesium presented, preferably to between 0.1 and 10 mol %.

5. A method according to at least one of claims 1 to 4, **characterised in that** the magnesium is employed in relation to the organic halide (R¹X) with an excess between 10 and 200%.

6. A method according to at least one of claims 1 to 5, **characterised in that** the protic amine (R'R"NH) and the organic halide (R¹X) are employed in the molar ratio of protic amine (R'R"NH) to organic halide (R¹X) between 0.7 and 1.5, preferably between 0.9 and 1.3, particularly preferably between 1.0 and 1.2.

7. A method according to at least one of claims 1 to 6, **characterised in that** in mixtures of ethers and hydrocarbons, organic halides (R¹X) are used that produce the desired hydrocarbon during the reaction.

8. A method according to at least one of claims 1 to 6, **characterised in that** in pure ethers, organic halides (R¹X) are used that form volatile gases during the reaction.

9. A method according to at least one of claims 1 to 8, **characterised in that**
in the organic halides (R¹X), the protic amines (R'R"NH), the Grignard compounds (R¹MgX) and the silyl fragments (R²R³R⁴Si), R¹, R², R³, R⁴, R' and R" are chosen independently of one another from:
- methyl, ethyl, n-propyl, iso-propyl, n-butyl, sec-butyl, tert-butyl, pentyl-, hexyl-, heptyl-, octyl-, cyclohexyl, phenyl and benzyl fragments, and wherein
- as regards organic halides (R¹X), methyl chloride or bromide, ethyl chloride or bromide, n- or iso-propyl chloride or bromide, n-, sec- or tert-butyl chloride or bromide, n-, iso-, sec- or tert-pentyl chloride or bromide, n-hexyl chloride or bromide, chlorobenzene or bromobenzene or benzyl chloride or bromide are employed, and wherein
- as regards protic amines (R'R"NH), dimethylamine, diethylamine, di-n-propylamine, di-iso-propylamine (DIPA), di-n-butylamine, di-sec-butylamine, di-tert-butylamine, dicyclohexylamine, N-tert-butyl-iso-propylamine, hexamethyldisilazane, piperidine and 2,2,6,6-tetramethylpiperidine (TMP) are employed.

10. A method according to claim 1, **characterised in that** compounds of the general formula MY are used as alkali-metal salts,
- wherein M is lithium and potassium, particularly preferably lithium,
- and wherein Y is selected from chloride, bromide, iodide or OZ,
- wherein OZ represents an alcoholate anion, and Z is chosen from an organic, branched or unbranched, saturated or unsaturated, aliphatic or aromatic carbon fragment that contains between one and 20 carbon atoms or is selected from R²R³R⁴Si, where R², R³ and R⁴ are as defined below,
- wherein fragments with one to 10 carbon atoms and R²R³R⁴Si are preferred,
- wherein methyl, ethyl, n-propyl, iso-propyl, n-butyl, sec-butyl, tert-butyl, tert-amyl, phenyl and R²R³R⁴Si are particularly preferred.

11. A method according to claim 10, **characterised in that** lithium chloride, lithium bromide, lithium methoxide, lithium ethoxide, lithium-n-propoxide, lithium-isopropoxide, lithium-tert-butoxide, lithium-tert-amoxide, lithium-trimethylsilyl oxide, lithium-tri-tert-butylsilyl oxide are used as alkali-metal salts.

12. A method according to claim 1, **characterised in that** an excess of 0 - 50% alkali salt is employed relative to the desired molar ratio of MY to R'R"NMgX.

13. A method according to claim 1, **characterised in that** a molar ratio of MY to R'R"NMgX ≥ 1 is striven for, wherein the excess of alkali salt (MY) to organic halide (R¹X) amounts to between 0 - 50%.

## Revendications

1. Procédé de préparation de mélanges constitués d'halogénures d'amido-magnésium, de formule générale R'R"NMgX, et de sels de métal alcalin (MY), **caractérisé en ce qu'**on met le magnésium et une amine protique (R'R"NH) dans un solvant ou un mélange de solvants organique(s) aprotique(s) et l'on ajoute à ce mélange un halogénure organique (R¹X), ou bien on met le magnésium dans un solvant ou un mélange de solvants organique(s) aprotique(s) et l'on y ajoute une amine protique (R'R"NH) et un halogénure organique (R¹X), soit à l'état de mélange, soit en parallèle à l'état isolé, le sel de métal alcalin étant préparé en même temps, étant entendu que :
- comme halogénures organiques (R¹X), on utilise les suivants : chlorure ou bromure de méthyle, chlorure ou bromure d'éthyle, chlorure ou bromure de n-propyle ou d'isopropyle, chlorure ou bromure de n-butyle, de sec-butyle ou de tertiobutyle, chlorure ou bromure de n-pentyle, d'isopentyle, de sec-pentyle ou de tertiopentyle, chlorure ou bromure de n-hexyle, chlorobenzène, bromobenzène, ou chlorure ou bromure de benzyle ;
- comme amines protiques (R'R"NH), on utilise les suivantes : diméthyl-amine, diéthyl-amine, di-n-propyl-amine, di-iso-propylamine (DIPA), di-n-butyl-amine, di-sec-butyl-amine, ditertiobutyl-amine, dicyclohexyl-amine, N-tertiobutyl-isopropyl-amine, hexaméthyl-disilazane, pipéridine ou 2,2,6,6-tetraméthyl-pipéridine (TMP) ;
- M représente lithium, sodium ou potassium ;
- et Y représente un ion choisi parmi les ions chlorure, bromure et iodure et les ions alcoolates symbolisés par OZ où Z représente une entité choisie parmi les fragments carbonés organiques, ramifiés ou non ramifiés, saturés ou insaturés, aliphatiques ou aromatiques, et comportant de 1 à 20 atomes de carbone, ou une entité choisie parmi les groupes de formule R²R³R⁴Si où R², R³ et R⁴ représentent des entités choisies, indépendamment les unes des autres, parmi les fragments organiques aliphatiques, cycliques, hétérocycliques ou aromatiques, saturés ou insaturés, ramifiés ou non ramifiés, fonctionnalisés ou non-fonctionnalisés, et comportant de 1 à 20 atomes de carbone.

2. Procédé conforme à la revendication 1, **caractérisé en ce que** le magnésium est utilisé sous forme de blocs, de copeaux, de râpures, de granulat ou de poudre de magnésium.

3. Procédé conforme à la revendication 2, **caractérisé en ce que** le magnésium est activé par addition d'iode, de 1,2-dibromo-méthane, de chlorure de triméthyl-silyle, d'une solution de Grignard (R¹MgX) préparée au préalable, ou de produit R'R"NMgX préparé au préalable, à l'état de mélange avec un sel de métal alcalin (MY), et de préférence, avec une solution de Grignard (R¹MgX) préparée au préalable, ou du produit R'R"NMgX préparé au préalable, à l'état de mélange avec un sel de métal alcalin (MY).

4. Procédé conforme à la revendication 3, **caractérisé en ce que** la quantité de solution de Grignard (R'MgX), ou de produit R'R"NMgX préparé au préalable, à l'état de mélange avec un sel de métal alcalin (MY), utilisée pour l'activation représente, par rapport à la quantité de magnésium mise en jeu, entre 0,01 et 50 %, en moles, et de préférence, entre 0,1 et 10 % en moles.

5. Procédé conforme à l'une au moins des revendications 1 à 4, **caractérisé en ce qu'**on utilise le magnésium en excès de 10 à 200 % par rapport à l'halogénure organique (R¹X).

6. Procédé conforme à l'une au moins des revendications 1 à 5, **caractérisé en ce qu'**on utilise l'amine protique (R'R"NH) et l'halogénure organique (R¹X) en un rapport molaire de l'amine protique (R'R"NH) à l'halogénure organique (R¹X) qui vaut entre 0,7 et 1,5, de préférence entre 0,9 et 1,3, et surtout entre 1,0 et 1,2.

7. Procédé conforme à l'une au moins des revendications 1 à 6, **caractérisé en ce qu'**on utilise, dans des mélanges d'éthers et d'hydrocarbures, des halogénures organiques (R¹X) qui produisent, au cours de la réaction, l'hydrocarbure voulu.

8. Procédé conforme à l'une au moins des revendications 1 à 6, **caractérisé en ce qu'**on utilise, dans des éthers purs, des halogénures organiques (R¹X) qui forment, au cours de la réaction, des gaz volatils.

9. Procédé conforme à l'une au moins des revendications 1 à 8, **caractérisé en ce que**, dans les halogénures organiques R¹X, les amines protiques R'R"NH, les composés de Grignard R¹MgX et les fragments silyle R²R³R⁴Si, les symboles R¹, R², R³, R⁴, R' et R" représentent des groupes choisis, indépendamment les uns des autres, parmi les groupes méthyle, éthyle, n-propyle, isopropyle, n-butyle, sec-butyle, tertiobutyle, pentyle, hexyle, heptyle, octyle, cyclohexyle, phényle et benzyle, étant entendu que :
- comme halogénures organiques (R¹X), on utilise les suivants : chlorure ou bromure de méthyle, chlorure ou bromure d'éthyle, chlorure ou bromure de n-propyle ou d'isopropyle, chlorure ou bromure de n-butyle, de sec-butyle ou de tertiobutyle, chlorure ou bromure de n-pentyle, d'isopentyle, de sec-pentyle ou de tertiopentyle, chlorure ou bromure de n-hexyle, chlorobenzène, bromobenzène, ou chlorure ou bromure de benzyle ;
- comme amines protiques (R'R"NH), on utilise les suivantes : diméthyl-amine, diéthyl-amine, di-n-propyl-amine, di-iso-propylamine (DIPA), di-n-butyl-amine, di-sec-butyl-amine, ditertiobutylamine, dicyclohexyl-amine, N-tertiobutyl-isopropyl-amine, hexaméthyl-disilazane, pipéridine ou 2,2,6,6-tetraméthyl-pipéridine (TMP).

10. Procédé conforme à la revendication 1, **caractérisé en ce que**, comme sels de métal alcalin, on utilise des composés de formule générale MY dans laquelle
- M représente lithium ou potassium, de préférence lithium ;
- et Y représente un ion choisi parmi les ions chlorure, bromure et iodure et les anions alcoolates symbolisés par OZ où Z représente une entité choisie parmi les fragments carbonés organiques, ramifiés ou non ramifiés, saturés ou insaturés, aliphatiques ou aromatiques, et comportant de 1 à 20 atomes de carbone, ou une entité choisie parmi les groupes de formule R²R³R⁴Si où R², R³ et R⁴ ont les significations indiquées plus haut, étant entendu
- que sont préférés les fragments comportant de 1 à 10 atomes de carbone et les groupes de formule R²R³R⁴Si,
- et que sont particulièrement préférés les groupes méthyle, éthyle, n-propyle, isopropyle, n-butyle, sec-butyle, tertiobutyle, tertioamyle et phényle et les groupes de formule R²R³R⁴Si.

11. Procédé conforme à la revendication 10, **caractérisé en ce que**, comme sels de métal alcalin, on utilise les suivants : chlorure de lithium, bromure de lithium, méthylate de lithium, éthylate de lithium, n-propylate de lithium, isopropylate de lithium, tertiobutylate de lithium, tertioamylate de lithium, triméthyl-silyl-oxy-lithium, tri(tertiobutyl)-silyl-oxy-lithium.

12. Procédé conforme à la revendication 1, **caractérisé en ce qu'**on utilise le sel de métal alcalin en excès de 0 à 50 % par rapport au rapport molaire de MY à R'R"NMgX voulu.

13. Procédé conforme à la revendication 1, **caractérisé en ce qu'**on vise un rapport molaire de MY à R'R"NMgX supérieur ou égal à 1, l'excès de sel de métal alcalin (MY) par rapport à l'halogénure organique (R¹X) valant de 0 à 50 %.
